(19) Europäisches Patentamt
European Patent Office
Office européen des brevets

(11) Numéro de publication: 0 192 521
B1

(12) # FASCICULE DE BREVET EUROPÉEN

(45) Date de publication du fascicule du brevet: 14.12.88

(51) Int. Cl.4: C 07 D 211/70, A 61 K 31/44

(21) Numéro de dépôt: 86400153.2

(22) Date de dépôt: 27.01.86

(54) **Dérivés de 3-phényl-tétrahydropyridine, procédé de préparation et utilisation en thérapeutique.**

(30) Priorité: 01.02.85 FR 8501410

(43) Date de publication de la demande: 27.08.86 Bulletin 86/35

(45) Mention de la délivrance du brevet: 14.12.88 Bulletin 88/50

(84) Etats contractants désignés: AT BE CH DE FR GB IT LI LU NL SE

(56) Documents cité:
FR-A-1 455 825
FR-A-2 496 099
US-A-4 263 438

JOURNAL OF THE CHEMICAL SOCIETY, CHEMICAL COMMUNICATIONS, no. 16, 20 août 1975, page 682; R.V. STEVENS et al.:"Acid-catalysed rearrangement of cyclobutylimines. A new synthesis of tetrahydropyridines"
Idem
JOURNAL OF MEDICINAL CHEMISTRY, vol. 24, no. 12, décembre 1981, pages 1475-1482, American Chemical Society; U. HACKSELL et al.: "3-Phenylpiperidines. Central dopamine-autoreceptor stimulating activity"

(73) Titulaire: LABORATOIRE L. LAFON Société anonyme dite:, 1 rue Georges Médéric, F-94701 Maisons- Alfort (FR)

(72) Inventeur: Lafon, Louis, 5 rue de l'Alboni, F-75016 Paris (FR)

(74) Mandataire: Clisci, Serge, S.A. FEDIT- LORIOT CONSEILS EN PROPRIETE INDUSTRIELLE 38, avenue Hoche, F-75008 Paris (FR)

EP 0 192 521 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

**Description**

La présente invention concerne, en tant que produits industriels nouveaux, des dérivés de 3-phényltétrahydropyridine. Elle concerne également le procédé de préparation et l'utilisation en thérapeutique de ces nouveaux dérivés.

Les nouveaux dérivés selon l'invention appartiennent à la famille des N-alkyl-3-phényl-1,2,5,6- et 1,4,5,6-tétrahydropyridines de fomules I et I' ci-après.

Un connaît deja, en tant que curiosité de laboratoire, la N-méthyl-3-phényl-1,2,5,6-tétrahydropyridine de l'article de R.V. STEVENS et al., Journal of the Chemical Society, Chemical Communications, No. 16, page 682, (1975), ce produit (qui est référencé composé A ci-après) état obtenu par réarrangement cyclique à partir d'une phényl-cyclobutylimine selon le mécanisme:

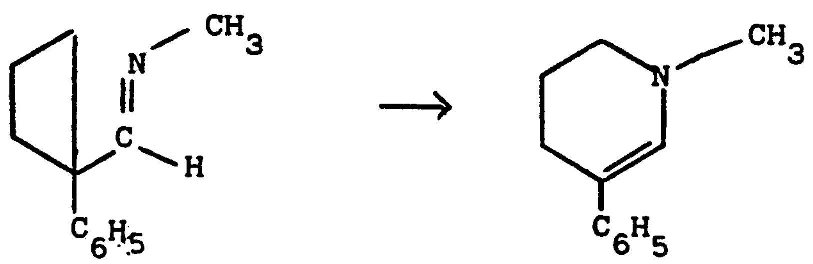

sans que ses éventuelles propriétés pharmacologiques soient signalées dans ledit article.

On connaît également de l'article de U. HACKSELL et al., J. Med. Chem., 24, 1475-1482 (1981) un procédé de préparation de N-alkyl-3-phénylpipéridines par réduction de 3-phénylpyridines en 3-phénylpipéridines puis N-alkylation, das lequel la réduction ne conduit pas aux dérivés 3-phényl-1,2,5,6-tétrahydropyridines.

On connaît également de FR-A-2 496 099 des dérivés de N-alkyl-3-(3-hydroxyphényl)-1,2,5,6-tétrahydropyridine présentés en tant que substances douées de propriétés agonistes ou antagonistes dopaminergiques.

On sait que dans US-A-4 263 438 des dérivés de N-alkyl- et N-benzyl-3-phényl-1,2,5,6-tétrahydropyridine, où le groupe phényle situé en position 3 du cycle tétrahydropyridine comporte deux substituants, ont éte présentés en tant que produits intermédiaires dans la synthèse de dérivés de 3-phénylpipéridine utiles en tant qu'agents analgésiques.

Un connaît enfin de FR-A-1 455 825 des dérivés de N-alkyl-4-(halogénophényl)-1,2,3,6-tétrahydropyridine présentés en tant que substances vermifuges.

Selon l'invention on préconise de nouveaux dérivés de N-alkyl-3-phényl-1,2,5,6- et/ou 1,4,5,6-tétrahydropyridine, qui sont structurellement différents des produits de l'art atérieur sus-visés, possèdent des propriétés intéressantes notamment sur le système nerveux central (SNC), agissent das l'organisme en tant qu'agents sédatifs, et ont l'avatage d'être dépourvus d'effets tératogènes néfastes.

Les nouveaux dérivés de 3-phényl-tétrahydropyridine selon l'invention sont caractérisés en ce qu'ils sont choisis parmi l'ensemble constitué par

(i) les N-alkyl-3-phényl-1,2,5,6- et 1,4,5,6-tétrahydropyridines répondant aux formules générales

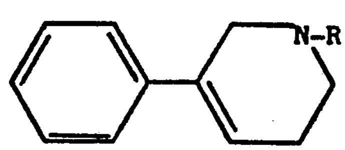

(I) ,

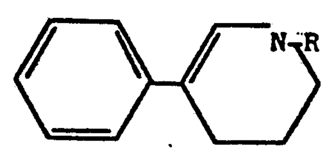

(I bis)

dans lesquelles R représente un groupe alkyle en $C_2$-$C_4$, et leurs mélanges; et,

(ii) leurs sels d'addition.

Parmi les groupes R qui conviennent selon l'invention on peut notamment citer les groupes $CH_2CH_3$, $CH_2CH_2CH_3$, $CH(CH_3)_2$, $CH_2CH_2CH_2CH_3$, $CH(CH_3)CH_2CH_3$, $CH_2CH(CH_3)_2$ et $C(CH_3)_3$, les groupes préférés étant $CH_2CH_3$, $CH(CH_3)_2$ et $C(CH_3)_3$. Les composés les plus intéressants sur le plan thérapeutique sont ceux dans lesquels R est $CH_2CH_3$ ou $CH(CH_3)_2$.

Par sels d'addition on entend ici, d'une part, les sels d'addition d'acide obtenus par réaction d'une base libre de formule I ou I bis avec des acides minéraux ou organiques et, d'autre part, les sels d'ammonium. Parmi les acides utilisables pour salifier lesdites bases, on peut notamment citer les acides chlorhydrique, bromhydrique, acétique, formique, propionique, oxalique, fumarique, maléique, succinique, benzoique, cinnamique,

mandélique, citrique, malique, tartrique, aspartique, glutamique, méthanesulfonique, p-toluènesulfonique. Parmi les composés permettant d'obtenir des sels d'ammonium on peut notamment citer $ICH_3$ et $ClCH_3$. D'une manière générale les sels d'addition d'acide sont préférés aux sels d'ammonium.

Eu égard au procédé de préparation décrit ci-après chaque dérivé de 3-phényl-tétrahydropyridine selon l'invention est un produit de formule I, de formule I bis ou un mélange des deux isomères 1,2,5,6-tétrahydro et 1,4,5,6-tétrahydro. Dans l'état actuel des connaissances la structure "1,2,5,6-tétrahydro" selon la formule I est celle qui, semble-t-il, prédomine dans la formule des composés selon l'invention. C'est pourquoi par commodité l'expression "dérivés de 3-phényl-1,2,5,6-tétrahydropyridine" désigne ci-après non seulement les composés de structure 1,2,5,6-tétrahydro, mais encore ceux de structure 1,4,5,6-tétrahydro et les mélanges des deux structures.

Un certain nombre de composés selon l'invention sont consignés de façon nullement limitative dans le tableau I ci-après.

Tableau I

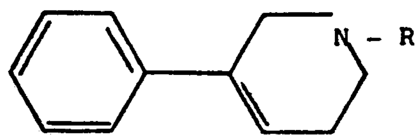

| Produit | N° de Code | R |
|---|---|---|
| Ex 1(a) | CRL 41 244 | $CH(CH_3)_2$ |
| Ex 2(a) | - | $C(CH_3)_3$ |
| Ex 3(b) | CRL 41 244 A | $CH(CH_3)_2$ |
| Ex 4(c) | CRL 41 244 B | $CH(CH_3)_2$ |
| Ex 5(b) | - | $C(CH_3)_3$ |
| Ex 6(a) | CRL 41 124 | $CH_2CH_3$ |

Notes:
(a) : chlorhydrate
(b) : fumarate
(c) : méthanesulfonate

Les produits selon l'invention peuvent être préparés selon une méthode connue en soi par application de mécanismes réactionnels classiques. Le procédé de synthèse, que l'on préconise ici, consiste à soumettre une N-alkyl-3-hydroxy-3-phénylpipéridine de formule

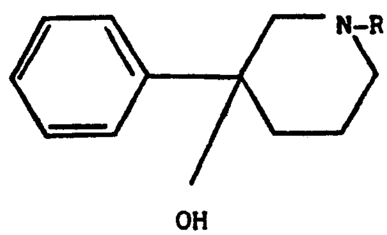

(II)

(où R est défini comme indiqué ci-dessus) à une réaction de déshydratation. De façon avantageuse cette réaction est réalisée en milieu acide, de préférence dans un mélange $CH_3COOH$-$CH_3COX$ (où X est un atome d'halogène, notamment F, Cl ou Br, et de préférence Cl).

Le meilleur mode de mise en oeuvre de ce procédé consiste à faire réagir un milieu réactionnel comprenant une N-alkyl-3-hydroxy-3-phénylpipéridine de formule II et un mélange $CH_3COOH$-$CH_3COCl$ (1 : 1) v/v, pendant au moins 1 h à la température de reflux dudit milieu réactionnel.

Les composés selon l'invention ont des propriétés intéressantes en thérapeutique. En particulier ils agissent sur le SNC, notamment en tant qu'agents sédatifs.

Selon l'invention on préconise une composition thérapeutique qui est caractérisée en ce qu'elle renferme, en association avec un excipient physiologiquement acceptable, au moins un composé de formule I ou I bis ou l'un de leurs sels d'addition, en tant que principe actif.

Bien entendu, dans une telle composition le principe actif, qui est choisi parmi l'ensemble constitué par les composés de formules I, I bis et leurs sels d'addition non toxiques, intervient en quantité pharmaceutiquement efficace.

Les produits préférés selon l'invention sont la N-isopropyl-3-phényl-1,2,5,6-tétrahydropyridine, la N-éthyl-3-phényl-1,2,5,6-tétrahydropyridine, la N-tertiobutyl-3-phényl-1,2,5,6-tétrahydropyridine et leurs sels d'addition, les produits les plus intéressants sur le plan thérapeutique étant la N-isopropyl-3-phényl-1,2,5,6-tétrahydropyridine et ses sels d'addition d'acide ainsi que la N-éthyl-3-phényl-1,2,4,5-tétrahydropyridine et ses

sels d'addition d'acide.

D'autres avantages et caractéristiques de l'invention se nont mieux compris à la lecture qui va suivre d'exemples de préparation et de résultats d'essais pharmacologiques, l'ensemble de ces éléments n'étant nullement limitatif mais donné à titre d'illustration.

**Préparation I**

Obtention du chlorhydrate de N-isopropyl-3-phényl-1,2,5,6-tétrahydropyridine.

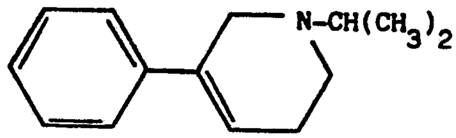

, HCl

(Exemple 1; N° 90 de code: CRL 41 244);

autre nomenclature: chlorhydrate de 1-isopropyl-3-phényl-1,2,5,6-tétrahydropyridine.

On chauffe au reflux pendant 2 h un mélange réactionnel comprenant 0,02 mole de chlorhydrate de N-isopropyl-3-hydroxy-3-phénylpipéridine, 50 ml d'acide acétique et 50 ml de chlorure d'acétyle. On évapore à sec, reprend le résidu d'évaporation à l'acétone et filtre le précipité formé. Par recristallisation du mélange acétone-éthanol (1 : 1) v/v on obtient le CRL 41 244 avec un rendement de 49 %.

$F_{inst.}$ = 160° C.

**Préparation II**

Obtention du chlorhydrate de N-isopropyl-3-phényl-1,2,5,6-tétrahydropyridine (CRL 41 244) par synthèse totale.

La synthèse totale est schématiquement représentée par le diagramme A ci-après.

a) Chlorhydrate de N-benzyl-3-hydroxy-3-phénylpipéridine

On dissout 50 g (0,222 mole) de chlorhydrate de N-benzyl-3-pipéridinone dans de l'eau, on ajoute de la soude jusqu'à pH 11, extrait à l'éther diéthylique, lave la phase éthérée à l'eau, sèche sur $MgSO_4$ et filtre. On coule dans le filtrat refroidi par un bain de glace et maintenu sous atmosphère d'azote, une solution (3 moles, 90 $cm^3$) de bromure de phénylmagnésium dans l'éther. Quand l'addition de $C_6H_5MgBr$ est terminée, on laisse le milieu réactionnel revenir à la température ambiante (15-20° C) et coule le milieu réactionnel dans 1 kg de glace. On recueille la phase éthérée par décantation, la lave à l'eau et la sèche sur $MgSO_4$. Après filtration pour écarter $MgSO_4$ on précipite le chlorhydrate attendu au moyen d'éthanol chlorhydrique. Par recristallisation du mélange acétone-éthanol (1 : 1) v/v on obtient 8,8 g (rendement: 13 %) de chlorhydrate de N-benzyl-3-hydroxy-3-phénylpipéridine.

Analyse
- % $Cl^-$ mesuré: 12,08 %
- % $Cl^-$ théorique: 11,70 %

b) Chlorhydrate de 3-hydroxy-3-phénylpipéridine

On dissout 8,8 g (0,029 mole) de chlorhydrate de N-benzyl-3-hydroxy-3-phénylpipéridine dans 400 ml de méthanol, ajoute 1,60 g de palladium sur charbon activé en suspension dans de l'éthanol anhydre et on met en contact avec $H_2$. On absorbe 700 ml d'hydrogène, puis filtre le milieu réactionnel et on évapore le filtrat à sec. On reprend le résidu d'évaporation avec de l'éther et filtre le précipité formé que l'on sèche. On obtient 4,5 g (rendement global des étapes a et b: 9,5 %) de chlorhydrate de 3-hydroxy-3-phénylpipéridine.

$F_{inst.}$ = 244° C (avec décomposition)

Analyse
- % $Cl^-$ mesuré: 16,86 %
- % $Cl^-$ théorique : 16,63 %

c) Chlorhydrate de N-isopropyl-3-hydroxy-3-phényl-pipéridine

On chauffe au reflux pendant 4 h un mélange réactionnel de 28,3 g (0,132 mole) de chlorhydrate de 3-hydroxy-3-phénylpipéridine, de 24,77 g (0,146 mole) d'iodure d'isopropyle, de 46,4 g (0,438 mole) de $CO_3Na_2$ et de 300 ml d'eau. On refroidit, extrait à l'éther, lave la phase éthérée et la sèche sur $MgSO_4$. On filtre puis à partir du filtrat on précipite le chlorhydrate attendu au moyen d'éthanol chlorhydrique. Par recristallisation du mélange acétone-éthanol (1 : 1) v/v on obtient 23 g (rendement: 68 %) de chlorhydrate de N-isopropyl-3-hydroxy-3-phénylpipéridine. $F_{inst.}$ = 194° C (avec décomposition).

d) CRL 41 244

On chauffe au reflux pendant 2 h un mélange de 10 g (0,039 mole) de chlorhydrate de N-isopropyl-3-hydroxy-3-phénylpipéridine, de 60 ml d'acide acétique et de 60 ml de chlorure d'acétyle. On évapore à sec, reprend le résidu d'évaporation avec de l'acétone et filtre le précipité formé. Par recristallisation du mélange acétone-éthanol (1 : 1) v/v on obtient 5 g (rendement: 54 %) de CRL 41 244.

$F_{inst.}$ = 160° C.

**Préparation III**

Obtention du chlorhydrate de N-tertiobutyl-3-phényl-1,2,5,6-tétrahydropyridine.
(Exemple 2)

En procédant comme indiqué à l'étape d) de la préparation II mais en remplaçant le chlorhydrate de N-isopropyl-3-hydroxy-3-phénylpipéridine par le chlorhydrate de N-tertiobutyl-3-hydroxy-3-phénylpipéridine, on obtient le chlorhydrate de N-tertiobutyl-3-phényl-1,2,5,6-tétrahydropyridine.

**Préparation IV**

Obtention du chlorhydrate de N-éthyl-3-phényl-1,2,5,6-tétrahydropyridine
(Exemple 6: N° de code: CRL 41 124)

On dissout 8,4 g (0,0348 mole) de chlorhydrate de N-éthyl-3-hydroxy-3-phénylpipéridine dans 50 ml d'acide acétique. On ajoute 50 ml de chlorure d'acétyle et porte au reflux pendant 2 h sous garde à $CaCl_2$. On évapore à sec et reprend le résidu d'évaporation au moyen d'acétate d'éthyle, puis filtre le précipité formé. Par recristallisation du mélange acétone-éthanol (1 : 1) v/v on obtient 4 g (rendement: 51 %) de CRL 41 124.

$F_{inst.}$ = 180° C.

On a résumé ci-après les résultats des essais neuropsychopharmcologiques qui ont été entrepris avec le CRL 41 244 (produit de l'exemple 1) et le CRL 41 124 (produit de l'exemple 6). Dans ces essais chacun de ces produits a été administré par voie intrapéritonéale en solution dans de l'eau distillée, à pH 6 (CRL 41 244) ou à pH 5 (CRL 41 124), sous un volume de 20 ml/kg chez la souris mâle, et sous un volume de 5 ml/kg chez le rat mâle.

**A - Essais relatifs au CRL 41 244 (exemple 1)**

I. TOXICITE

Chez la souris mâle par voie i.p. la DL-50 du CRL 41 244 est de l'ordre de 90 mg/kg environ, et la DL-50 (dose maximale non mortelle) est de l'ordre de 30 mg/kg.

II. COMPORTEMENT GLOBAL ET REACTIVITES

Des lots de six animaux sont observés avant, puis 0,25 h, 0,50 h, 1 h, 2 h, 3 h et 24 h après l'administration de CRL 41 244. On constate chez la souris

à la dose de 0,125 mg/kg:
- une hypothermie (maximum: - 0, 9° C) pendat 0, 5 h;

à la dose de 0,5 mg/kg:
- une sédation (2 animaux sur 6) 0,25 h après administration,
- une diminution de la respiration (2/6) et de la réaction de peur (3/6) 0,25 h après administration,
- une hypothermie (maximum: - 1,9° C) pendant plus de 1 h;

à la dose de 2 mg/kg:
- une sédation pendant 1 h environ,
- une diminution de la respiration (à 0,15 h) et de la réaction de peur (pendant 1 h),
- une hypothermie (maximum: - 1,7° C) pendant plus de 1 h; et,

à la dose de 8 mg/kg:
- une sédation 0,15 h après administration durant 0,5 à 1 h,
- une diminution de la réaction de peur (pendant 1 h) et de la réaction au toucher (pendant 1 h),
- une mydriase modérée pendant 0,5 h, et
- une dyspnée pendant 1 h.

III. ACTION SUR LA TEMPERATURE RECTALE

La température rectale des souris (6 animaux par dose) est notée toutes les 30 minutes pendant 3 h, après l'administration du CRL 41 244. On constate que dès la dose de 0,032 mg/kg, le CRL 41 244 provoque une hypothermie qui atteint son intensité maximale entre 0,5 et 1 h et qui a une durée de 2 h; l'effet maximum est observé à la dose de 0,5 mg/kg.

IV. INTERACTION AVEC L'APOMORPHINE

1) chez la souris

Le CRL 41 244 est administré à des lots de 6 souris 30 minutes avant l'injection sous-cutanée de 1 ou 16 mg/kg d'apomorphine. On observe que, à la dose de 8 mg/kg, le CRL 41 244 antagonise l'hypothermie induite par l'apomorphine, sans modifier l'attitude de verticalisation et les stéréotypies, et qu'aux doses plus faibles (0,125 mg/kg, 0,5 mg/kg et 2 mg/kg) il provoque une augmentation de l'hypothermie due à l'apomorphine, alors que le CRL 41 244 administré seul est hypothermisant aux doses de 0,25 mg/kg, 0,5 mg/kg et 2 mg/kg et à un degré moindre à la dose de 8 mg/kg (voir résultats donnés ci-dessus).

2) chez le rat

Le CRL 41 244 est administré à des lots de 6 rats 0,5 h avant l'injection sous-cutanée de 0,5 mg/kg d'apomorphine. On observe que le CRL 41 244 ne modifie pas les stéréotypies induites par l'apomorphine.

V. INTERACTION AVEC L'AMPHETAMINE

L'amphétamine (2 mg/kg) est injectée par voie intrapéritonéale à des lots de 12 rats, 30 minutes après l'administration de CRL 41 224. On constate que, à la dose de 4 mg/kg, le CRL 41 244 diminue modérément des stéréotypies amphétaminiques.

VI. INTERACTION AVEC LA RESERPINE

Quatre heures après l'injection intrapéritonéale de 2,5 mg/kg de réserpine, des lots de 6 souris reçoivent le CRL 41 244. On note que le CRL 41 244 ne modifie pas l'hypothermie et le ptôsis réserpiniques.

VII. INTERACTION AVEC L'OXOTREMORINE

Le CRL 41 244 est administré à des lots de 6 souris 0,5 h avant l'injection intrapéritonéale de 0,5 mg/kg d'oxotrémorine.

1) Action sur la température

On constate que le CRL 41 244, qui utilisé seul a un effet hypothermisant, ne modifie pas l'action hypothermisante de l'oxotrémorine.

2) Action sur les tremblements

On constate que, aux doses de 2 mg/kg et 8 mg/kg, le CRL 41 244 diminue faiblement l'intensité des tremblements induits par l'oxotrémorine.

3) Action sur les symptômes cholinergiques périphériques

On observe que le CRL 41 244 ne modifie pratiquement pas les signes de stimulation cholinergique périphérique induits par l'oxotrémorine.

VIII. ACTION SUR LE TEST DES QUATRE PLAQUES, LA TRACTION ET L'ELECTROCHOC

Le test est pratiqué sur des lots de 10 souris, 30 minutes après l'administration de CRL 41 244.

On constate que, aux doses de 2 et 8 mg/kg, le CRL 41 244 diminue modérément le nombre de passages punis. Il ne provoque pas d'incoordination motrice, ne modifie pas les effets convulsivants et létailx de l'électrochoc.

IX. ACTION SUR LA MOTILITE SPONTANEE

0,5 h après avoir reçu le CRL 41 244, les souris (6 par dose, 12 témoins) sont placées en actimètre où leur motilité est enregistrée pendant 30 minutes.

Dès la dose de 0,032 mg/kg, le CRL 41 244 diminue l'activité motrice spontanée de la souris. L'intensité maximale de l'effet est atteinte rapidement (0,25 mg/kg) et se maintient à un niveau proche de 50 % de diminution pour les doses supérieures (comprises entre 0,25 mg/kg et 8 mg/kg).

X. ACTION SUR L'AGRESSIVITE INTERGROUPES

Après avoir séjourné pendant 3 semaines dans chacune des moitiés d'une cage séparées par une cloison opaque, des groupes de 3 souris reçoivent le CRL 41 244. Une demi-heure plus tard, les deux groupes d'une même cage sont réunis par retrait de la cloison et on note le nombre de combats qui surviennent en 10 minutes. On constate que, dès la dose de 0,25 mg/kg, le CRL 41 244 diminue fortement l'agressivité intergroupes; cet effet a tendance à disparaître à la plus forte dose utilisée (2 mg/kg).

XI. ACRION VIS-A-VIS DE QUELQUES COMPORTEMENTS PERTURBES PAR PAR DIVERS AGENTS.

1) Motilité réduite par habituation à l'enceinte.

Après 18 heures de séjour dans les actimètres, les souris (6 par dose, 12 témoins) reçoivent le CRL 41 244. Elles sont aussitôt replacées dans leurs enceintes respectives et, une demi-heure plus tard, on enregistre leur motilité pendant 30 minutes. On constate que le CRL 41 244 n'entraîne pas une reprise de l'activité motrice de

la souris habituée à son enceinte.

2) Motilité réduite par agression hypoxique.

0,5 h après avoir reçu le CRL 41 244, les souris (10 par dose, 20 témoins) sont soumises à une anoxie hypobare [dépression de 600 mmHg (i. e. environ 8 x $10^4$ Pa) en 90 secondes; puis détente en 45 secondes 7, puis elles sont placées en actimètre où leur motilité est enregistrée pendant 10 minutes.

On observe que le CRL 41 244. dès la dose de 0,125 mg/kg, entraîne une diminution de la récupération motrice chez la souris dont la motilité a été déprimée à la suite d'un bref séjour dans une enceinte sous pression réduite.

3) Anoxie asphyxique.

Des lots de 10 souris reçoivent le CRL 41 244, 30 minutes avant l'administration intrapéritonéale de 32 mg/kg de triiodoéthylate de gallamine (agent curarisant de référence). On observe que le CRL 41 244, dès la dose de 0,125 mg/kg, augmente significativement le délai d'apparition des convulsions et de la mort consécutives à l'anoxie asphyxique provoquée par un curarisant.

XII. INTERACTION AVEC LE BARBITAL

Une demi-heure après l'administration de CRL 41 244 des lots de 10 souris reçoivent une injection intrapéritonéale de barbital (220 mg/kg). On constate que le CRL 41 244 ne modifie pas la durée du sommeil barbiturique.

XIII. ACTION SUR LE "DESESPOIR COMPORTEMENTAL"

Une demi-heure après avoir reçu le CRL 41 244, des lots de 6 souris sont placés dans un bécher rempli d'eau sur une hauteur de 6 cm. On note la durée totale d'immobilité entre la 2ème et la 6ème minutes suivant l'immersion. On observe que le CRL 41 244 ne semble pas modifier la durée d'immobilité de la souris placée en immersion forcée.

XIV. CONCLUSIONS

Les résultats des essais donnés ci-dessus mettent en évidence que le CRL 41 244 agit en tant qu'agent sédatif.

Les essais cliniques ont permis de déterminer que la dose quotidienne à administrer per os à l'homme adulte est comprise entre 1 mg et 6 mg pour le CRL 41 244. En particulier on a obtenu d'excellents résultats cliniques en administrant per os 2 à 3 comprimés ou gélules (renfermant chacun 1 à 2 mg de CRL 41 244) par jour.

**B. Essais relatifs au CRL 41 124 (exemple 6)**

Les protocoles opératoires sont ceux donnes ci-dessus pour le CRL 41 244.

I. TOXICITE

Chez la souris mâle par voie i.p. la DL-0 du CRL 41 124 est de l'ordre de environ 50 mg/kg, et la DL-30 (dose létale pour 30 % des animaux) est de l'ordre de environ 60 mg/kg.

II. COMPORTEMENT GLOBAL ET REACTIVITES

On observe chez la souris

à la dose de 0,25 mg/kg:
- aucune modification nette du comportement et des réactivités par rapport aux témoins.

à la dose de 1 mg/kg:
- une sédation 1 h après administration, et
- une hypothermie (maximum: - 1° C, 30 minutes après administration durant pendant 1 h;

à la dose de 4 mg/kg:
- une sédation pendant 1 h environ,
- une polypnée 0,5 h après administration,
- une mydriase 0,5 h après administration, et

à la dose de 16 mg/kg:
- une sédation pendant 3 h,
- une polypnée pendant 1 h,
- une piloérection,
- une diminution de la réactivité au toucher et du tonus musculaire,
- une mydriase modérée 0,5 h après administration, et
- une hypothermie (-1,3° C 0,5 h après administration) durant pendat 2 h.

III. INTERACTION AVEC L'APOMORPHINE

1) chez la souris

On observe que, aux doses de 1 mg/kg, 4 mg/kg et 16 mg/kg, le CRL 41 124 antagonise l'hypothermie induite par l'apomorphine sans modifier l'attitude de verticalisation et les stéréotypies.

2) chez le rat

On observe que le CRL 41 124 ne modifie pas les stéréotypies induites par l'apomorphine.

IV. INTERACTION AVEC L'AMPHETAMINE

On constate que le CRL 41 124 ne modifie pas les stéréotypies induites par l'amphétamine.

V. INTERACTION AVEC LA RESERPINE

On observe que le CRL 41 124 ne modifie pas l'hypothermie réserpinique mais qu'il diminue modérément le ptôsis à la dose de 16 mg/kg.

VI. INTERACTION AVEC L'OXOTREMORINE

1) Action sur la température

On constate que le CRL 41 124, qui utilisé seul a un effet hypothermisant, antagonise l'action hypothermisante de l'oxotrémorine aux doses de 4 et 16 mg/kg.

2) Action sur les tremblements

On constate que le CRL 41 124 ne modifie pas les tremblements induits par l'oxotrémorine.

3) Action sur les symptômes cholinergiques phériphériques

On observe que le CRL 41 124 ne modifie pratiquement pas les signes de stimulation cholinergiques périphériques induits par l'oxotrémorine.

VII. ACTION SUR LE TEST DES QUATRE PLAQUES, LA TRACTION ET L'ELECTROCHOC.

On constate que le CRL 41 124 n'entraîne pas une augmentation du nombre de passages punis. Il ne provoque pas d'incoordination motrice, ne modifie pas les effets convulsivants mais aggrave, à forte dose (16 mg/kg) les effets létaux de l'électrochoc.

VIII. ACTION SUR LA MOTILITE SPONTANEE

On observe que, dès la dose de 0,01 mg/kg, le CRL 41 124 diminue l'activité motrice spontaée de la souris.

IX. ACTION SUR L'AGRESSIVITE INTERGROUPES

On constate que, aux doses de 0,06 et 0,25 mg/kg, le CRL 41 124 diminue le nombre de combats.

X. ACTION VIS-A-VIS DE QUELQUES COMPORTEMENTS PERTURBES PAR DIVERS AGENTS.

1) Motilité réduite par habituation à l'enceinte

On constate que le CRL 41 124 n'entraîne pas une reprise de l'activite motrice de la souris habituée à son enceinte.

2) Motilité réduite par agression hypoxique

On observe que le CRL 41 124, dès la dose de 0,25 mg/kg, entraîne une diminution de la récupération motrice chez la souris dont la motilité a été déprimée à la suite d'un bref séjour das une enceinte sous pression réduite.

3) Anoxie asphyxique

On observe que le CRL 41 124 ne modifie pas le délai d'apparition des convulsions et de la mort consécutives à l'anoxie asphyxique provoquée par un curarisant.

XI. INTERACTION AVEC LE BARBITAL

On constate que le CRL 41 124 ne modifie pratiquement pas la durée du sommeil barbiturique.

XII. ACTION SUR LE "DESESPOIR COMPORTEMENTAL"

On observe que le CRL 41 124 ne modifie pas la durée d'immobilité de la souris placée en immersion forcée.

XII. CONCLUSIONS

Les résultats des essais donnés ci-dessus mettent en évidence que le CRL 41 124 agit en tant qu'agent sédatif et présente des effets de type antidépresseur.

**C. Essais comparatifs**

Une étude tératogène a été entreprise chez la lapine (Fauve de Bourgogne pesant environ 2900 g avant gestation; les femelles de la variété Fauve de Bourgogne ont en général une portée de 2 à 10 petits). Le premier jour de la gestation (i.e. le jour où le mâle est introduit dans la cage des femelles) les femelles sont réparties en lots de 10 animaux par dose et produits à tester et en un lot de 15 animaux pour contrôle. Les lapines reçoivent quotidiennement par gastrogavage 0 (lot contrôle), 1 mg/kg et 5 mg/kg des produits à tester,

du 5è au 18é jour de la gestation. Une césarienne est effectuée le 28è jour de la gestation afin de compter:

(i) le nombre de lapines présentant dans leur portée au moins une malformation foetale, et
(ii) le nombre total de foetus présentant une ou plusieurs malformations.

Les résultats des essais comparatifs ont été consignés dans le tableau II ci-après. Ils mettent en évidence le fait que les produits selon l'invention sont dépourvus des effets tératogènes néfastes du produit de comparaison A décrit par STEVENS et al. précité.

Selon l'invention on préconise l'utilisation d'une substace choisie parmi les composés de formules I et I bis et leurs sels d'addition non-toxiques pour l'obtention d'un médicament sédatif destiné à une utilisation en thérapeutique humaine, notamment vis-à-vis des excitations.

**Tableau II**

**Etude teratogène**

| Produit | No. de code | Dose (mg/kg) | TF (a) | FM (b) Nombre | FM (b) % | NF (c) | MF (d) Nombre | MF (d) % |
|---|---|---|---|---|---|---|---|---|
| Contrôle | - | 0 | 15 | 0 | 0 | 72 | 0 | 0 |
| Ex 1 | CRL 41 244 | 1 | 10 | 0 | 0 | 52 | 0 | 0 |
| Ex 1 | CRL 41 244 | 5 | 10 | 0 | 0 | 48 | 0 | 0 |
| Ex 2 | - | 1 | 10 | 0 | 0 | 47 | 0 | 0 |
| Ex 2 | - | 5 | 10 | 0 | 0 | 54 | 0 | 0 |
| Ex 6 | CRL 41 124 | 1 | 10 | 0 | 0 | 47 | 0 | 0 |
| Ex 6 | CRL 41 124 | 5 | 10 | 0 | 0 | 55 | 0 | 0 |
| A(e) | - | 1 | 10 | 1 | 10 | 49 | 5 | 10,2 |
| A(e) | - | 5 | 10 | 2 | 20 | 52 | 9 | 17,3 |

Notes
(a) TF = nombre total de femelles par groupe
(b) FM = femelles présentant das leur portée au moins une malformation foetale
(c) NF = nombre total de foetus
(d) MF = foetus présentant une ou plusieurs malformations
(e) produit de comparaison, à savoir le chlorhydrate de N-méthyl-3-phényl-1,2,5,6-tétrahydropyridine, décrit par STEVENS et al., Journal of the Chemical Society, Chemical Communications No. 16, page 682 (1975)

**Diagramme A**

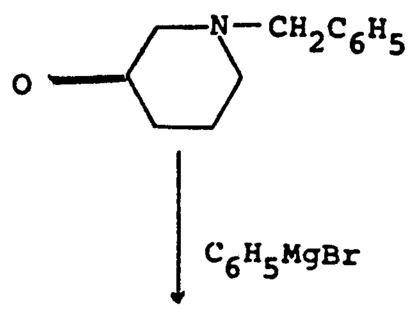

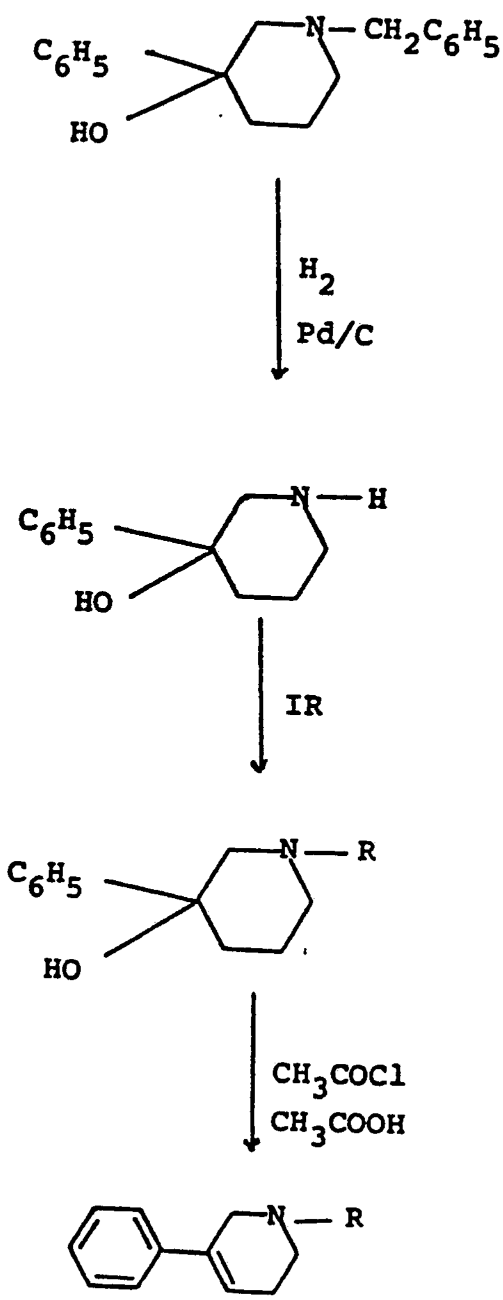
H2
Pd/C
IR
CH3COCl
CH3COOH

**Revendications** pour les états contractants: BE, CH, DE, FR, GB, IT, LI, LU, NL, SE

1. Dérivé de 3-phényl-tétrahydropyridine, caractérisé en ce qu'il est choisi parmi l'ensemble comprenant

(i) les N-alkyl-3-phényl-1,2,5,6- et 1,4,5,6-tétrahydropyridines répondant aux formules générales

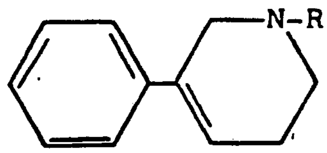

(I)

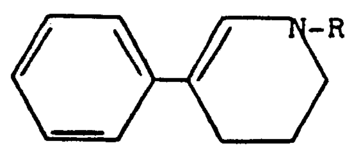

(I bis)

das lesquelles R représente un groupe alkyle en $C_2$-$C_4$ et leurs mélanges; et,

(ii) leurs sels d'addition.

2. Dérivé selon la revendication 1, caractérisé en ce que R représente $CH_2CH_3$, $CH(CH_3)_2$ ou $C(CH_3)_3$.

3. N-Isopropyl-3-phényl-1,2,5,6-tétrahydropyridine et ses sels d'addition.

4. N-Ethyl-3-phényl-1,2,5,6-tétrahydropyridine et ses sels d'addition.

5. Composition thérapeutique, caractérisée en ce qu'elle renferme, en association avec un excipient physiologiquement acceptable, au moins un composé N-alkyl-3-phényl-tétrahydropyridine ou l'un de ses sels d'addition selon la revendication 1, en tant qu'ingrédient actif.

6. Composition selon la revendication 5, caractérisée en ce qu'elle renferme un composé choisi parmi l'ensemble comprenant la N-isopropyl-3-phényl-1,2,5,6-tétrahydropyridine, la N-éthyl-3-phényl-1,2,5,6-tétrahydropyridine, et leurs sels d'addition en tant qu'ingrédient actif.

7. Procédé de préparation d'une N-alkyl-3-phényl-tétrahydropyridine de formule I ou I bis selon la revendication 1, caractérisé en ce que l'on soumet à une réaction de déshydratation un dérivé de N-alkyl-3-hydroxy-3-phénylpipéridine de formule

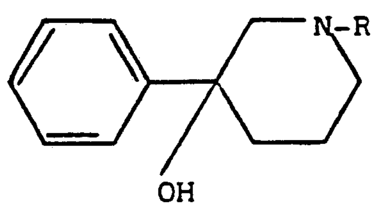

(II)

où R est un groupe alkyle en $C_2$-$C_4$, au moyen d'un mélange $CH_3COOH$-$CH_3COX$ (où X est F, Cl ou Br) pendant au moins 1 h à la température de reflux du milieu réactionnel.

8. Procédé selon la revendication 7, caractérisé en ce que le moyen deshydratant est un mélange $CH_3COOH$-$CH_3COCl$ (1 : 1) v/v.

9. Utilisation d'une substance selon la revendication 1, pour l'obtention d'un médicament sédatif destiné à une utilisation en thérapeutique humaine.

**Revendications** pour l'état contractant AT

1. Procédé de préparation d'un dérivé de 3-phényl-tétrahydropyridine choisi parmi l'ensemble comprenant

(i) les N-alkyl-3-phényl-1,2,5,6- et 1,4,5,6-tétrahydropyridines répondant aux formules générales

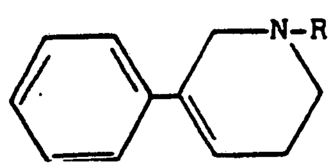

(I)

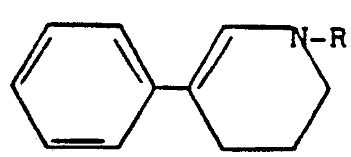

(I bis)

dans lesquelles R représente un groupe alkyle en $C_2$-$C_4$, et leurs mélanges; et,

(ii) leurs sels d'addition;

ledit procédé étant caractérisé en ce que l'on soumet à une réaction de déshydratation un dérivé de N-alkyl-3-hydroxy-3-phénylpipéridine de formule

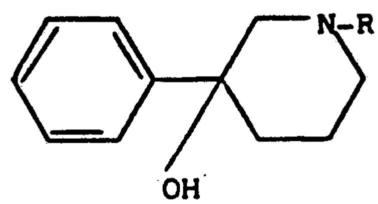

(II)

où R est un groupe alkyle en $C_2$-$C_4$, au moyen d'un mélange $CH_3COOH$-$CH_3COX$ (où X est F, Cl ou Br) pendant au moins 1 h à la température de reflux du milieu réactionnel.

2. Procédé selon la revendication 1, caractérisé en ce que le moyen déshydratant est un mélange $CH_3COOH$-$CH_3COCl$ (1 : 1) v/v.

**Claims** for the contracting stages : BE, CH, DE, FR, GB, IT, LI, LU, NL, SE

1. A 3-phenyl-tetrahydropyrine derivative selected from the group comprising

(i) the N-alkyl-3-phenyl-1,2,5,6- and 1,4,5,6-tetrahydropyridines of the formulae:

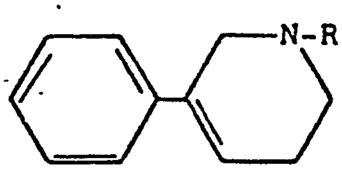

(I) ;

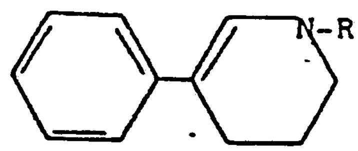

(I bis)

wherein R represents a $C_2$-$C_4$ alkyl group, and, their mixtures; and,

(ii) addition salts thereof.

2. A derivative according to claim 1, in which R represents $CH_2CH_3$, $CH(CH_3)_2$ or $C(CH_3)_3$.

3. N-Isopropyl-3-phenyl-1,2,5,6-tetrahydropyridine and addition salts thereof.

4. N-Ethyl-3-phenyl-1,2,5,6-tetrahydropyridine and addition salts thereof.

5. A therapeutical composition comprising, in association with a physiologically acceptable excipient, a pharmaceutically effective amount of a compound of the formula I or I bis according to claim 1 or one of its non-toxic addition salts.

6. A therapeutical composition according to claim 5, which comprises a pharmaceutically effective amount of a compound selected from the group comprising N-isopropyl-3-phenyl-1,2,5,6-tetrahydropyridine, N-ethyl-3-phenyl-1,2,5,6-tetrahydropyridine and non-toxic addition salts thereof.

7. A method for preparing a N-alkyl-3-phenyl-tetrahydropyridine of the formula I or I bis according to claim 1, comprising subjecting a N-alkyl-3-hydroxy-3-phenylpiperidine derivative of the formula

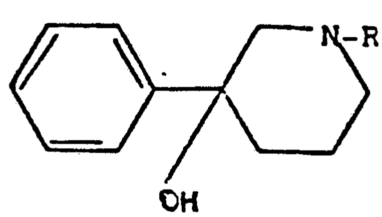

(II)

wherein R is a $C_2$-$C_4$ alkyl group, to a dehydratation reaction by means of a $CH_3COOH$-$CH_3COX$ mixture, in wich N is F, Cl or Br, for at least 1 hour at the reflux temperature of the reaction medium.

8. A method according to claim 7, in which the dehydratation means is a $CH_3COOH$-$CH_3COCl$ (1 : 1) v/v mixture.

9. The use of a substance according to claim, for preparing a sedative medicament destined to a use in human therapy.

**Claims** for the contracting state: AT

1. A method for preparing a 3-phenyl-tetrahydropyridine derivative selected from the group comprising

(i) the N-alkyl-3-phenyl-1,2,5,6- and 1,4,5,6-tetrahydropyridines of the formulae:

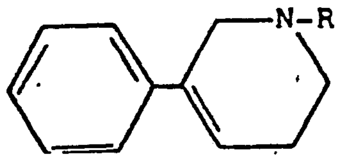

(I)

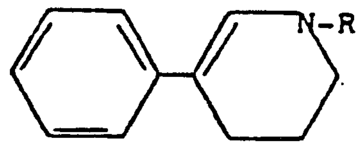

(I bis)

wherein R represents a $C_2$-$C_4$ alkyl group, and, their muixtures; and,

(ii) addition salts thereof,

said method comprising subjecting a N-alkyl-3-hydroxy-3-phenylpiperidine derivative of the formula

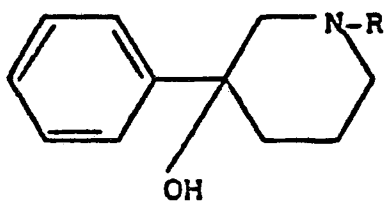

(II)

wherein R is a $C_2$-$C_4$ alkyl group, to a dehydratation reaction by means of a $CH_3COOH$-$CH_3COX$ mixture, in which N is F, Cl or Br, for at least 1 hour at the reflux temperature of the reaction medium.

2. A method according to claim 1, in which the dehydratation mean is a $CH_3COOH$-$CH_3COCl$ (1 : 1) v/v mixture.

**Patentansprüche** für die Vertragsstaaten: BE, CH, DE, FR, GB, IT, LI, LU, NL, SE

1. Derivat des 3-Phenyl-tetrahydropyridins,
<u>dadurch gekennzeichnet,</u>
daß es ausgewählt ist aus der Gruppe, die

(i) die N-Alkyl-3-phenyl-1,2,5,6 und 1,4,5,6-tetrahydropyridine entsprechend den allgemeinen Formeln

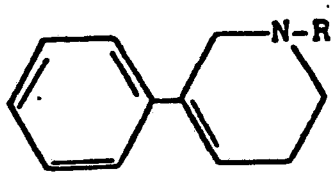

(I)

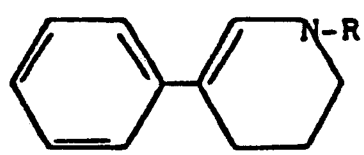

(I bis)

in denen R eine $C_2$-$C_4$- Alkylgruppe oder deren Mischungen darstellt, sowie

(ii) deren Additionssalze umfaßt.

2. Derivat gemäß Patentanspruch 1,
<u>dadurch gekennzeichnet,</u>
daß R $CH_2CH_3$, $CH(CH_3)_2$ oder $C(CH_3)_3$ bedeutet.

3. N-Isoprophyl-3-phenyl-1,2,5,6-tetrahydropyridin und dessen Additionssalze.

4. N-Ethyl-3-phenyl-1,2,5,6-tetrahydropyridin und dessen Additionssalze.

5. Therapeutische Zusammensetzung,
<u>dadurch gekennzeichnet,</u>
daß sie zusammen mit einem physiologisch annehmbaren Träger wenigstens eine Verbindung aus der Gruppe von N-Alkyl-3-phenyl-tetrahydropyridine oder eines von deren Additionssalzen gemäß Patentanspruch 1 als Wirkstoff enthält.

6. Zusammensetzung gemäß Patentanspruch 5,
dadurch gekennzeichnet,
daß sie als Wirkstoff eine Verbindung ausgewählt aus der Gruppe von N-Isopropyl-3-phenyl-1,2,5,6-tetrahydropyridin, N-Ethyl-3-phenyl-1,2,5,6-tetrahydropyridin und deren Additionssalze enthält.

7. Verfahren zur Herstellung eines N-Alkyl-3-phenyl-tetrahydropyridins der Formeln I oder I bis gemäß Patentanspruch 1,
dadurch gekennzeichnet,
daß man ein Derivat eines N-Alkyl-3-hydroxy-3-phenylpiperidins der Formel

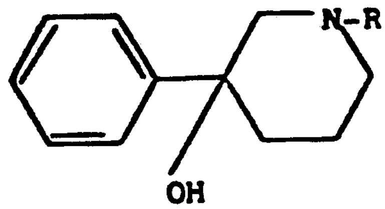

(II)

in der R eine $C_2$-$C_4$-Alkylgruppe ist, mittels einer Mischung von $CH_3COOH/CH_3COX$ (X = F, Cl oder Br) während wenigstens einer Stunde unter Rückflußtemperatur der Reaktionsmischung einer Dehydratisierung unterwirft.

8. Verfahren gemäß Anspruch 7,
dadurch gekennzeichnet,
daß das dehydratisierende Mittel eine Mischung aus $CH_3COOH$ und $CH_3COCl$ im Volumenverhältnis 1 : 1 ist.

9. Verwendung einer Substanz gemäß Patentanspruch 1 zur Darstellung eines sedierenden Medikaments, das zum Gebrauch in der Humantherapie bestimmt ist.

**Patentansprüche** für den Vertragsstaat AT

1. Verfahren zur Herstellung eines Derivats von einem 3-Phenyl-tetrahydropyridin ausgewählt aus der Gruppe, die umfaßt

(i) die N-Alkyl-3-phenyl-1,2,5,6 und 1,4,5,6-tetrahydropyridine gemäß den allgemeinen Formel

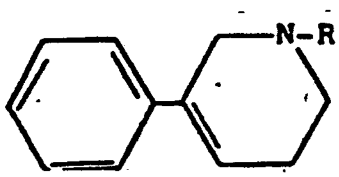

(I)

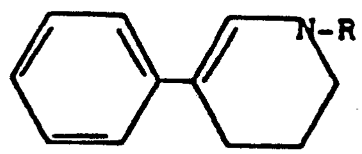

(I bis)

in denen R eine $C_2$-$C_4$-Alkylgruppe oder deren Mischungen bedeutet sowie
(ii) deren Additionssalze umfaßt, wobei das Verfahren

dadurch gekennzeichnet,
daß man ein Derivat von einem N-Alkyl-3-hydroxy-3-phenylpiperidin der Formel

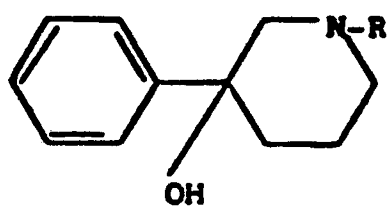

(II),

in der R eine $C_2$-$C_4$-Alkylgruppe darstellt, mittels einer Mischung von $CH_3COOH/CH_3COX$ (X = F, Cl oder Br) während mindestens einer Stunde bei der Rückflußtemperatur des Reaktionsmediums einer Dehydratisierung unterwirft.

2. Verfahren gemäß Patentanspruch 1,
dadurch gekennzeichnet,
daß das dehydratisierende Mittel eine Mischung aus $CH_3COOH$ und $CH_3COCl$ im Volumenverhältnis 1 : 1 ist.